# EUROPEAN PATENT SPECIFICATION

(11) **EP 2 538 863 B1**
(45) Date of publication and mention of the grant of the patent: **03.07.2019**
(21) Application number: 11714405.5
(22) Date of filing: 22.02.2011
(51) Int. Cl.: A61B 8/08, A61B 5/00, A61B 18/02, A61B 17/00, A61B 18/00, A61B 90/00, A61B 18/14, A61B 34/20

(54) **INTERVENTIONAL ABLATION DEVICE WITH TISSUE DISCRIMINATING CAPABILITY**
EINGRIFFSABLATIONSVORRICHTUNG MIT GEWEBEUNTERSCHEIDUNGSMÖGLICHKEIT
DISPOSITIF CHIRURGICAL D'ABLATION CAPABLE DE DIFFÉRENCIER LES TISSUS

(30) Priority: 26.02.2010 EP 10154770
(43) Date of publication of application: 02.01.2013
(73) Proprietor: Koninklijke Philips N.V., 5656 AE Eindhoven (NL)
(72) Inventor: HENDRIKS, Bernardus, Hendrikus, Wilhelmus, NL-5656 AE Eindhoven (NL); LUCASSEN, Gerhardus, Wilhelmus, NL-5656 AE Eindhoven (NL); NACHABÉ, Rami, NL-5656 AE Eindhoven (NL); BIERHOFF, Waltherus, Cornelis, Jozef, NL-5656 AE Eindhoven (NL); DESJARDINS, Adrien, Emmanuel, NL-5656 AE Eindhoven (NL)
(74) Representative: van Velzen, Maaike Mathilde
(86) International application number: PCT/IB2011/050727
(87) International publication number: WO 2011/104664

(56) References cited:
- WO-A2-01/74252
- WO-A2-2008/131302
- US-A1- 2005 234 437
- US-A1- 2006 173 359
- US-A1- 2008 119 846
- US-B1- 6 511 478

## Description

### FIELD OF THE INVENTION

The present invention relates to an interventional ablation device which may be used for example for ablating tumorous tissue within a body of a patient. Furthermore, the present invention relates to an interventional ablation needle, to a computer program element enabling to control an ablation procedure and a computer readable medium with such computer program element.

### BACKGROUND OF THE INVENTION

In oncology ablation of tumors is a common procedure especially in cases where resection of the tumor is difficult or almost impossible. For example, in the liver when there are plural tumor sites, complete removal may not be possible when these sites are present in different parts of the liver. What is typically done then is that part of the liver containing the major tumor sites is removed while the remaining part of the liver, also containing tumor sites, is treated by a needle ablation procedure. Therein, one or more needles may be positioned within the tumorous tissue for example by using image guidance based on for example previously or simultaneously acquired ultrasound or computer tomography images.

Several types of ablation techniques are known. For example, radio frequency (RF) ablation may be used during intervention to treat tumorous tissue. Typically, a RF ablation needle produces a high frequency alternating current between 100 kHz and 500 kHz. Ions are agitated by the induced electromagnetic field and due to friction this motion is converted into heat. The heat in turn may induce cell death and hence may result in the destruction of tumor cells.

However, heat propagation may be difficult to predict because it may depend strongly on morphology of the heated tissue and whether for instance blood vessels acting as a heat sink are present. Therefore, it may be almost impossible for a surgeon to tell whether a tumor has been completely treated, especially because such ablation progression generally is not visible under normal ultrasound vision.

WO 2008/023321 A2 describes an interventional device for RF ablation for use in a RF electrical and/or magnetic field especially of a MR imaging system comprises an ablation catheter which is preferably trackable or can be guided or visualized in the image generated by the MR imaging system by means of a micro-coil. However, the alternative of using magnetic resonance imaging (MRI) for guidance of the ablation needle could make the intervention very costly and impractical because then during the surgical intervention such surgery equipment needs to be available. Another similar device is described in WO 01/74252.

### SUMMARY OF THE INVENTION

There may be a need for an interventional ablation device and a computer program element allowing simple and cost-effective monitoring of an ablation procedure.

Such need may be met by the subject-matter of the independent claims, which define the scope of the invention. Advantageous embodiments are described in the dependent claims.

According to a first aspect of the present invention, an interventional ablation device comprises an ablation needle with an elongated body, an ablation element and at least one sensor element. Therein, the device is adapted for detecting physiological information of tissue surrounding an ablation site based on measurement values provided by the sensor.

According to a second aspect of the present invention, an ablation needle with an elongated body, an ablation element and at least one sensor element is proposed. The sensor is adapted for providing measurement values enabling a detecting of physiological information of tissue surrounding an ablation site. Preferably, the needle comprises two or more sensors at opposite sides of the ablation element.

According to a third aspect of the present invention a computer program element is adapted for enabling, when executed on a computer, to control the following processes during an ablation procedure: acquiring measurement values provided by a sensor arranged on an elongated body of an interventional ablation device; and providing physiological information based on the acquired measurement values. Preferably, an ablation element provided on the body may be controlled based on the physiological information or the physiological information may be displayed to a user.

A gist of the present invention may be seen in the idea to integrate one or more sensors into the elongated body of an interventional ablation device which sensor(s) allows to detect physiological information of tissue which is treated using the ablation element for example during a surgical ablation intervention. From the detected physiological information, it may then be possible to discriminate a type of tissue adjacent to the sensor, i.e., whether the tissue surrounding the body of the ablation device adjacent to the sensor is for example normal healthy tissue, tumorous tissue or ablated tissue. Such tissue discrimination information may then be provided to a surgeon or may be used to automatically control an ablation procedure. For example, knowing a volume and geometry of a tumor enclosed within healthy tissue for example by preceding acquisition of computer tomography information and furthermore knowing a precise location of the ablation device with respect to the tumor as well as a precise location of the sensor with respect to the ablation element of the ablation device, an ablation procedure may be precisely controlled allowing to completely destroy the tumor without unnecessarily affecting adjacent healthy tissue.

The sensor may be for example an optical sensor possibly connected to or comprising a light source and a light detector. The sensor may then be adapted to provide measurement values based on light reflected by adjacent tissue. Advantageously, the sensor may be adapted for measuring a reflectance spectrum of the reflected light. From such reflectance spectrum, the type of tissue may be derived. Other types of optical sensors using optical techniques such as fluorescence detection, two-photon spectroscopy, Raman spectroscopy, differential path length spectroscopy or diffuse optical tomography may be used as well for detecting the physiological information of the adjacent tissue. Furthermore, the sensor may be adapted for microscopic sensing like using fiber bundle approach, scanning optical coherence tomography or scanning fiber technology.

Advantageously, at least two sensors are arranged at opposing sides of the ablation element, preferably along a line parallel to the longitudinal axis of the elongated body. Such two sensors may be arranged adjacent to the ablation element and at a predetermined distance apart from the ablation element. Having such two sensors arranged at opposing sides of the ablation element may allow for monitoring an ablation progression in both opposing directions away from the ablation element and parallel to the elongated body.

Advantageously, a plurality of sensors is arranged at opposing sides of the ablation element. The sensors may be arranged along a line parallel to the longitudinal axis of the elongated body and may be spaced apart from each other at predetermined distances. By monitoring the measurement values provided by each of the spaced apart sensors, an ablation progression may be monitored and the ablation process may be stopped as soon as the entire tumorous tissue has been ablated and before an excessive amount of healthy tissue is affected.

In further embodiments, the ablation device may additionally comprise a controller for automatically controlling the ablation element based on the detected physiological information. For example, such controlling may be based on measurement values of specific sensors out of a plurality of sensors indicating that during the ablation procedure, tissue adjacent to the sensor changes optical properties due to a transition from tumorous tissue to ablated tissue while neighboring sensors detect healthy tissue or a transition from healthy tissue to ablated tissue. Such information may then be used to stop the ablation procedure.

Additionally, the controller may take into account additional information on tissue at the ablation site obtained in pre-operative data acquisition such as e.g. information on a geometry or volume of tumorous tissue obtained by e.g. a preceding MRI analysis.

Furthermore, the ablation device may comprise an imaging device for acquiring a plurality of measurement values provided by one or more sensors in different orientations of the respective sensor and generating there from a two-dimensional image. Furthermore, having generated a plurality of two-dimensional images at different locations of the respective sensor, a three-dimensional image may be generated using e.g. tomographical techniques.

It is to be noted that aspects and embodiments of the present invention are described herein with reference to different subject-matters. In particular, some embodiments are described with reference to the interventional ablation device and its components such as particularly an ablation needle whereas other features are described with reference to specifically using or controlling such interventional ablation device. However, a person skilled in the art will gather from the above and the following description that, unless other notified, in addition to any combination of features belonging to one type of subject-matter also any combination between features relating to different subject-matters is considered to be disclosed with this application.

### BRIEF DESCRIPTION OF THE DRAWINGS

Features and advantages of the present invention will be further described with reference to specific embodiments as shown in the accompanying figures but to which the invention shall not be limited.
Fig. 1 schematically shows an interventional ablation device according to an embodiment of the present invention.
Figs. 2 to 4 schematically show a progression of an ablation procedure using an ablation device according to an embodiment of the present invention.
Figs. 5 to 7 show examples of reflectance spectra measured by an optical sensor as it may be used in an ablation device according to an embodiment of the present invention.

The features shown in the drawings are schematic only and are not to scale. Throughout the figures, similar features are indicated with similar reference signs.

### DETAILED DESCRIPTION OF EMBODIMENTS

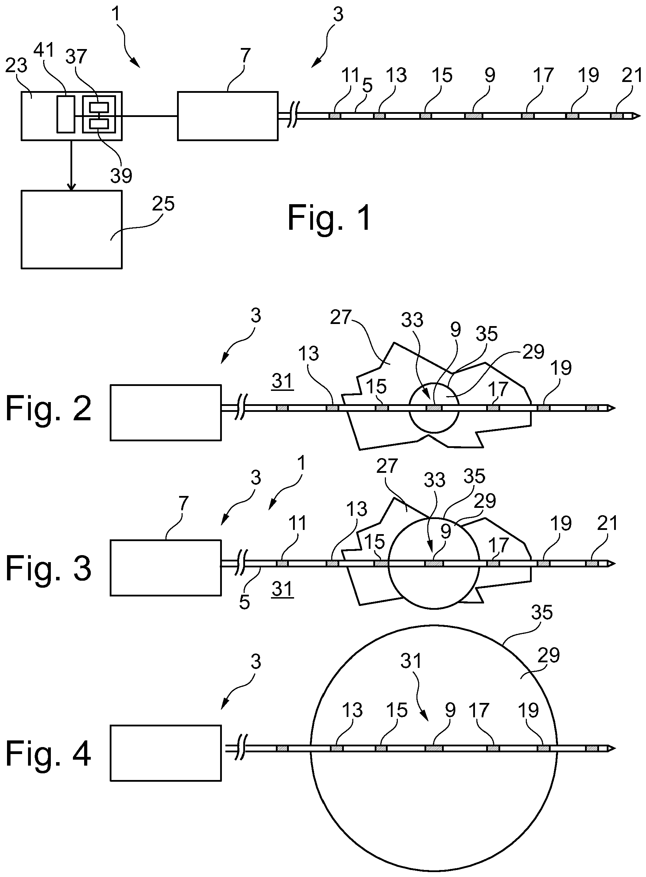

Fig. 1 shows an interventional ablation device 1 according to an embodiment of the present invention. The ablation device 1 may be used for example to ablate, i.e. remove or destroy, e.g. malicious tissue such as tumorous tissue enclosed by healthy tissue.

The ablation device 1 comprises an ablation needle 3 having an elongated body 5 and a handle 7. The elongated body 5 has a small diameter of e.g. between 22 and 11 gauge i.e. 0.72 and 3.05 mm and a length of e.g. between 100 and 300 mm or even more preferred between 120 and 250 mm. Furthermore, the elongated body 5 has a pointed tip at a distal end thereof thereby enabling to introduce the ablation needle easily into a patient's tissue.

An ablation element 9 is arranged on the body 5. The ablation element 5 is arranged in a region close to the distal end of the body 5 but spaced apart from this distal end. For example, the ablation element may be arranged at a distance between 5 and 100 mm away from the distal end of the body 5.

At opposite sides along the body 5, a plurality of sensors 11, 13, 15, 17, 19, 21 are arranged on the body 5. The sensors are spaced apart from each other and from the ablation element 9 at distances of e.g. between 1 and 50 mm or even more preferred between 1 and 10 mm.

The ablation needle 3 may be provided as a disposable product. Such disposable needle may be connected to further components of the ablation device. For example, the needle 3 may be connected to a controller console 23 which may acquire measurement values from the sensors provided on the needle and may control the ablation element provided on the needle. The needle may be disposed and replaced after each operation.

The ablation element 9 may be a radio frequency ablation (RFA) element adapted to emit energy by producing a high frequency alternating current in a range of 100 kHz to 500 kHz. Such high frequency energy may be absorbed by ions comprised in the adjacent tissue and due to an agitation of these ions, the tissue may be effectively heated. The heat may induce cell death. Accordingly, when the ablation needle is inserted into a patient's tissue such that its ablation element 9 is located within tumorous tissue, such tumorous tissue may be locally heated and the tumor cells may be destroyed.

Alternatively, the ablation element may rely on other ablation principles such as e.g. cryoablation. In cryoablation, adjacent tissue is cooled down and tumor cells may be killed by icing.

As schematically shown in the sequence of Figs. 2 to 4, the ablation needle 3 may be inserted into tumorous tissue 27 enclosed by normal healthy tissue 31 such that the ablation element 9 is located approximately in the center of the tumorous tissue 27. A correct positioning of the ablation needle 3 may be monitored e.g. using external imaging means such as ultrasound imaging or computer tomography. After starting an ablation procedure, the ablation element 9 heats or cools adjacent tissue within an ablation site 31. The ablation volume 35 including ablated tissue 29 around an ablation site 33 in which biological cells have been killed due to excessive heat or icing grows with continuing ablation progress.

Conventionally, a surgeon has not been able to monitor the progress of the ablation, i.e. to monitor whether the lesion generated during the ablation procedure has already destroyed the entire tumor or not and whether healthy tissue is started to be damaged. As the heat/icing propagation is difficult to predict because it typically depends strongly of the morphology of the adjacent tissue and whether for instance blood vessels are present, the surgeon had to rely on his experience and frequently not the entire tumor has been destroyed or, on the other side, excessive healthy tissue has been damaged during an ablation procedure.

In order to overcome such deficiencies, the ablation device 1 proposed herein comprises a multiplicity of sensors 11, 13, 15, 17, 19, 21 arranged along a longitudinal direction on the elongated body 5 on both sides of the ablation element 9. Each of the sensors 11-21 may measure parameter values which may be used to indicate physiological information concerning adjacent tissue 27, 29, 31 such as indicating whether the adjacent tissue is healthy tissue 31, tumorous tissue 27 or ablated tissue 29.

For this purpose, the sensors 11-21 may be provided as optical sensors adapted for measuring a reflectance spectrum of light reflected by the adjacent tissue. The optical sensor may comprise an optical fiber (not shown in the figures for clarity reasons). A distal end of the optical fiber may be arranged at the distal end of the body 5 thus forming a local sensor 11 - 21. A proximal end of the optical fiber may be connected to a control console 23. In the console 23, a light source 37 such as an LED and a light detector 39 may be provided. Light coming from the light source 37 may be coupled into the optical fiber at the proximal end and may propagate towards the distal end where it exits the optical fiber and may illuminate adjacent tissue. Light that is back-reflected towards this fiber may then be captured by the fiber and guided to the detector 39 at the fiber proximal end. Using such system comprising a light source 37, optical fiber and a light detector 39, the reflectance spectrum may be acquired. Particularly, the reflectance spectrum may be measured in the visible and/or near infrared range.

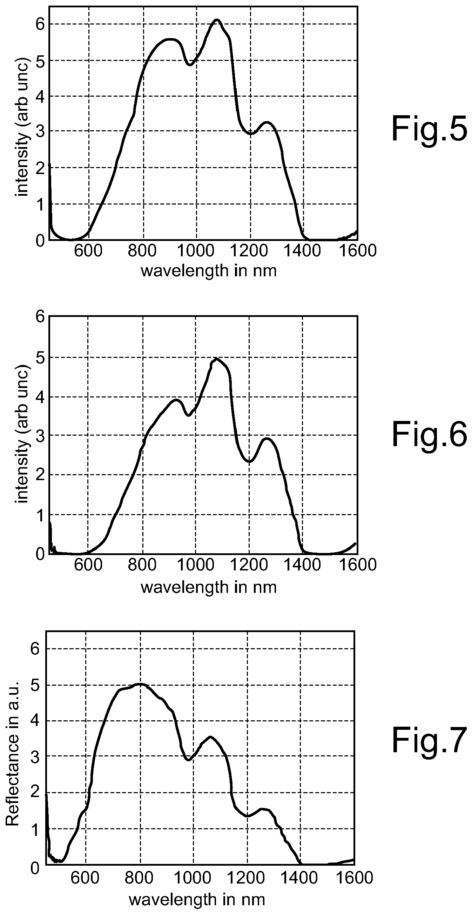

Figs. 5 to 7 show reflectance spectra as they may be detected by the detector comprised in one of the sensors 11-21. Such spectra as acquired with reflectance spectroscopy may have typical characteristics depending on the type of tissue 27, 29, 31 and in particular of its physiological properties. For example, the reflectance spectrum shown in Fig. 5 indicates normal healthy liver tissue 31. The reflectance spectrum shown in Fig. 6 represents tumorous liver tissue 27. The reflectance spectrum shown in Fig. 7 represents ablated liver tissue 29. Accordingly, from the measurements provided by the sensors 11-21 and the reflectance spectra obtained therewith, physiological information of tissue surrounding an ablation side 33 may be derived.

Coming back to Figs. 2 to 4, an ablation control scheme may be explained. At the beginning of the ablation process, the RF ablation element 9 is provided with electrical energy which is then transformed to RF energy heating a volume 35 around the ablation site 33 adjacent to the ablation element 9. At that point in time, none of the sensors 11-21 arranged equidistant along the body 5 at both sides of the ablation element 9 detects ablated tissue 29. The sensors 15, 17 closest to the ablation element 9 are lying within the tumor and therefore detect a reflectance spectrum representing tumorous tissue 27. The sensors 11, 13, 19, 21 further away from the ablation element 9 are positioned outside the tumor and therefore detect a reflectance spectrum indicating normal healthy tissue 31.

With progressing ablation process, the volume 35 of the ablation site 33 increases. As shown in Fig. 3, the ablated volume 35 reaches the innermost sensor 15, 17 after a while but does not yet reach the outer sensors 13, 19. This indicates that the ablation is not sufficient yet. In Fig. 4, the ablated volume 35 has also reached the sensors 13, 19. These sensors 13, 19 have originally detected a reflectance spectrum indicating healthy tissue 31 and now measure a reflectance spectrum indicating ablated tissue 29. Based on such monitoring result, the ablation device may detect that the ablated volume 29 covers the entire tumor and that the ablation process may be stopped. Such information may either be indicated to a surgeon via a display 25 connected to the console 23 of the interventional ablation device 1 or may be used internally in the console 23 in order to automatically stop an energy supply to the ablation element 9 thereby stopping the ablation process.

In the above described embodiment, physiological information is acquired along a one-dimensional cross-section of the tumor using the sensors 11-21 arranged along the body 5 of the ablation device 1. Since the tumor may be irregular, i.e. larger in a direction perpendicular to the longitudinal direction of the elongated body 5, it may be advantageous to combine the information provided by the ablation device 1 with pre-operative data acquired prior to the insertion of the ablation device by using other imaging modalities such as e.g. CT or MRI. From such pre-operative data, the dimension of the tumor in various directions may be deduced. The information that the tumor is smaller in a direction parallel to the longitudinal direction of the inserted elongated body 5 than in a direction perpendicular thereto may be used to correct or adapt the controlling of the ablation procedure. As the dimension of the elongated body 5 and the position of the sensors 11-21 relative to the ablation element 9 are known, the size of the tumor along the elongated body 5 may be measured. Using the size perpendicular to the body's 5 longitudinal direction coming from the pre-operative data, the ablation process may be suitably controlled and stopped as soon as the ablated volume 29 has reached a size that is larger than the largest dimension of the tumor.

According to a further embodiment, the ablation device 1 further comprises an imaging device 41 possibly comprised in the console 23. During an ablation process, the ablation needle 3 may be rotated about a longitudinal axis of the body 5. Thereby, although the sensors 11-21 are positioned only along one dimension and each of the sensors is adapted to acquire a measurement value at one adjacent point in space only, a two-dimensional imaging may be enabled by acquiring a plurality of measurement values using the sensors when arranged in different orientations. Accordingly, a 2D image containing physiological information on adjacent tissue may be acquired from measurement values of the rotated sensors 11-21. Using tomographic algorithms such as e.g. those that have been developed for diffuse optical tomography (DOT), even three-dimensional imaging may be possible by acquiring a plurality of 2D images generated at different locations of the sensors 11-21.

Finally, it is to be noted that instead of or additional to measuring reflectance spectra, the sensors 11-21 may be adapted for measuring other parameters indicative of physiological information using for example fluorescence detection, two-photon spectroscopy, Raman spectroscopy, differential path length spectroscopy or diffuse optical tomography. Furthermore, sensors may be capable of microscopic sensing like using fiber bundle approach, scanning optical coherence tomography or scanning fiber technology. Furthermore, apart from optical sensors, also other sensors like temperature sensors, PH sensors, stiffness sensors or ultrasound transducers may be arranged along the elongated body 5 of the ablation device 1 in order to complement the optical sensors 11-21. The ultrasound technique can be combined with optical methods like photoacoustic detection.

It should be noted that the term "comprising" and similar does not exclude other elements or steps and that the indefinite article "a" does not exclude a plurality of items. Also elements described in association with different embodiments may be combined. It should be furthermore noted that reference signs in the claims shall not be construed as limiting the scope of the claims.

### List of reference signs:

- 1: Ablation device
- 3: Ablation needle
- 5: Elongated body
- 7: Handle
- 9: Ablation element
- 11: Sensor
- 13: Sensor
- 15: Sensor
- 17: Sensor
- 19: Sensor
- 21: Sensor
- 23: Console
- 25: Display
- 27: Tumorous tissue
- 29: Ablated tissue
- 31: Normal tissue
- 33: Ablation volume
- 35: Ablation site
- 37: light source
- 39: light detector
- 41: imaging device

## Claims

1. An interventional ablation needle usable for an ablation device, the needle (3) comprising an elongated body (5) having a distal end, an ablation element (9) arranged in a region close to the distal end of the body (5) and spaced apart from the distal end, and at least two optical sensors (11, 13, 15, 17, 19, 21) arranged on the body (5) adjacent to, and spaced apart from, the ablation element (9) and at opposing sides of the ablation element (9) along a line parallel to the longitudinal axis of the elongated body (5), the optical sensors being adapted for providing measurement values enabling a detecting of physiological information of tissue (27, 29, 31) surrounding an ablation site (33).

2. An interventional ablation device (1), comprising:
an interventional ablation needle (3) according to claim 1,
wherein the device (1) is adapted for detecting physiological information of tissue (27, 29, 31) surrounding an ablation site (33) based on measurement values provided by the sensors (11, 13, 15, 17, 19, 21).

3. The device of claim 2, wherein the sensors (11, 13, 15, 17, 19, 21) are connected to a light source (37) and a light detector (39) and wherein the sensors (11, 13, 15, 17, 19, 21) are adapted for providing measurement values based on light reflected by the tissue (27, 29, 31).

4. The device of claim 2, wherein the device (1) is adapted for measuring a reflectance spectrum of the light reflected by the tissue (27, 29, 31).

5. The device of claim 2, wherein the device (1) is adapted for at least one of fluorescence detection, two-photon spectroscopy, Raman spectroscopy, differential path length spectroscopy, diffuse optical tomography and microscopic sensing.

6. The device of claim 2, additionally comprising at least one of a temperature sensor, a PH-sensor, a stiffness sensor and an ultrasound sensor.

7. The device of claim 2, wherein the device comprises a controller console (23) for controlling the ablation element (9) based on the detected physiological information.

8. The device of claim 7, wherein the controller console (23) is further adapted to control the ablation element (9) taking into account additional information on tissue at the ablation site obtained in pre-operative data acquisition.

9. The device of claim 2, wherein the device comprises an imaging device (41) for acquiring a plurality of measurement values provided by a sensor in different orientations of the sensor and generating a 2D image from the acquired measurement values.

10. The device of claim 9, wherein the imaging device (41) is adapted to generate a 3D image from a plurality of 2D images generated at different locations of the sensor.

11. A computer program element enabling, when executed on a computer, to control the following processes during an ablation procedure:
acquiring measurement values provided by a sensor (11, 13, 15, 17, 19, 21) arranged on an elongated body (5) of an interventional ablation needle (3) according to claim 1;
providing physiological information of tissue (27, 29, 31) surrounding an ablation site (33) based on the acquired measurement values.

12. The computer program element of claim 11, wherein the controlled processes further comprises:
- controlling an ablation element (9) provided on the body (5) based on the physiological information.

13. A computer readable medium with a computer program element according to claim 12.

## Patentansprüche

1. Eingriffsablationsnadel, die für eine Ablationsvorrichtung verwendet werden kann, wobei die Nadel (3) einen verlängerten Körper (5) mit einem distalen Ende, einem Ablationselement (9), das in einer Region nahe dem distalen Ende des Körpers (5) angeordnet und vom distalen Ende beabstandet ist, und mindestens zwei optische Sensoren (11, 13, 15, 17, 19, 21) umfasst, die auf dem Körper (5), benachbart zu und beabstandet von dem Ablationselement (9) und auf gegenüberliegenden Seiten des Ablationselements (9) entlang einer Linie parallel zur Längsachse des verlängerten Körpers (5) angeordnet sind, wobei die optischen Sensoren ausgelegt sind, um Messwerte bereitzustellen, die einen Nachweis von physiologischer Information von Gewebe (27, 29, 31), das eine Ablationsstelle (33) umgibt, ermöglichen.

2. Eingriffsablationsvorrichtung (1), umfassend:
eine Eingriffsablationsnadel (3) nach Anspruch 1, wobei die Vorrichtung (1) ausgelegt ist, um physiologische Information von Gewebe (27, 29, 31), das eine Ablationsstelle (33) umgibt, nachzuweisen, basierend auf Messwerten, die von den Sensoren (11, 13, 15, 17, 19, 21) bereitgestellt werden.

3. Vorrichtung nach Anspruch 2, wobei die Sensoren (11, 13, 15, 17, 19, 21) mit einer Lichtquelle (37) und einem Lichtdetektor (39) verbunden sind, und wobei die Sensoren (11, 13, 15, 17, 19, 21) ausgelegt sind, um Messwerte basierend auf Licht, das durch das Gewebe (27, 29, 31) reflektiert wird, bereitzustellen.

4. Vorrichtung nach Anspruch 2, wobei die Vorrichtung (1) ausgelegt ist, um ein Reflexionsspektrum des Lichts, das vom Gewebe (27, 29, 31) reflektiert wird, zu messen.

5. Vorrichtung nach Anspruch 2, wobei die Vorrichtung (1) für mindestens eines von Fluoreszenznachweis, Zweiphotonenspektroskopie, Raman-Spektroskopie, Differenzweglängenspektroskopie, diffuser optischer Tomographie und Mikroskopmessen ausgelegt ist.

6. Vorrichtung nach Anspruch 2, zusätzlich umfassend mindestens eines von einem Temperatursensor, einem PH-Sensor, einem Steifigkeitssensor und einem Ultraschallsensor.

7. Vorrichtung nach Anspruch 2, wobei die Vorrichtung eine Steuerkonsole (23) umfasst, um das Ablationselement (9) basierend auf der nachgewiesenen physiologischen Information zu steuern.

8. Vorrichtung nach Anspruch 7, wobei die Steuerkonsole (23) weiter ausgelegt ist, um das Ablationselement (9) unter Berücksichtigung von zusätzlicher Information über Gewebe an der Ablationsstelle, erhalten bei der präoperativen Datenerfassung, zu steuern.

9. Vorrichtung nach Anspruch 2, wobei die Vorrichtung eine Bildgebungsvorrichtung (41) umfasst, um eine Vielzahl von Messwerten zu erfassen, die von einem Sensor in verschiedenen Ausrichtungen des Sensors bereitgestellt werden, und ein 2D-Bild aus den erfassten Messwerten zu erzeugen.

10. Vorrichtung nach Anspruch 9, wobei die Bildgebungsvorrichtung (41) ausgelegt ist, um ein 3D-Bild aus einer Vielzahl von 2D-Bildern zu erzeugen, die an verschiedenen Stellen des Sensors erzeugt wurden.

11. Computerprogrammelement, das, wenn es auf einem Computer ausgeführt wird, ermöglicht, die folgenden Prozesse während eines Ablationsverfahrens zu steuern:
Erfassen von Messwerten, die von einem Sensor (11, 13, 15, 17, 19, 21) bereitgestellt werden, der auf einem verlängerten Körper (5) einer Eingriffsablationsnadel (3) nach Anspruch 1 angeordnet ist;
Bereitstellen von physiologischer Information von Gewebe (27, 29, 31), das eine Ablationsstelle (33) umgibt, basierend auf den erfassten Messwerten.

12. Computerprogrammelement nach Anspruch 11, wobei die gesteuerten Prozesse weiter Folgendes umfassen:
- Steuern eines Ablationselements (9), das auf dem Körper (5) bereitgestellt ist, basierend auf der physiologischen Information.

13. Computerlesbares Medium mit einem Computerprogrammelement nach Anspruch 12.

## Revendications

1. Aiguille d'ablation chirurgicale pouvant être utilisée pour un dispositif d'ablation, l'aiguille (3) comprenant un corps allongé (5) ayant une extrémité distale, un élément d'ablation (9) disposé dans une région proche de l'extrémité distale du corps (5) et espacée de l'extrémité distale, et au moins deux capteurs optiques (11, 13, 15, 17, 19, 21) disposés sur le corps (5) adjacents à, et espacés de, l'élément d'ablation (9) et sur des côtés opposés de l'élément d'ablation (9) le long d'une ligne parallèle à l'axe longitudinal du corps allongé (5), les capteurs optiques étant adaptés à fournir des valeurs de mesure permettant une détection d'informations physiologiques du tissu (27, 29, 31) entourant un site d'ablation (33).

2. Dispositif d'ablation chirurgical (1), comprenant :
- une aiguille d'ablation chirurgicale (3) selon la revendication 1,
- dans lequel le dispositif (1) est adapté à détecter des informations physiologiques du tissu (27, 29, 31) entourant un site d'ablation (33) basé sur des valeurs de mesures fournies par les capteurs (11, 13, 15, 17, 19, 21).

3. Dispositif selon la revendication 2, dans lequel les capteurs (11, 13, 15, 17, 19, 21) sont reliés à une source de lumière (37) et un détecteur de lumière (39) et dans lequel les capteurs (11, 13, 15, 17, 19, 21) sont adaptés à fournir des valeurs de mesure sur la base de la lumière réfléchie par le tissu (27, 29, 31).

4. Dispositif selon la revendication 2, dans lequel le dispositif (1) est adapté à mesurer un spectre de réflectance de la lumière réfléchie par le tissu (27, 29, 31).

5. Dispositif selon la revendication 2, dans lequel le dispositif (1) est adapté pour au moins l'une de la détection de fluorescence, de la spectroscopie à deux photons, de la spectroscopie Raman, de la spectroscopie de longueur de trajet différentielle, de la tomographie optique diffuse et de la détection microscopique.

6. Dispositif selon la revendication 2, comprenant en outre au moins l'un d'un capteur de température, d'un capteur de pH, d'un capteur de rigidité et d'un capteur à ultrasons.

7. Dispositif selon la revendication 2, dans lequel le dispositif comprend une console de dispositif de commande (23) pour commander l'élément d'ablation (9) sur la base des informations physiologiques détectées.

8. Dispositif selon la revendication 7, dans lequel la console de dispositif de commande (23) est en outre adaptée à commander l'élément d'ablation (9) en prenant en considération des informations supplémentaires sur le tissu au niveau du site d'ablation obtenues dans l'acquisition de données avant l'opération.

9. Dispositif selon la revendication 2, dans lequel le dispositif comprend un dispositif d'imagerie (41) pour acquérir une pluralité de valeurs de mesure fournies par un capteur dans différentes orientations du capteur et générer une image 2D à partir des valeurs de mesure acquises.

10. Dispositif selon la revendication 9, dans lequel le dispositif d'imagerie (41) est adapté à générer une image 3D à partir d'une pluralité d'images 2D générées à différents emplacements du capteur.

11. Élément de programme informatique permettant, lorsqu'il est exécuté sur un ordinateur, de commander les processus suivants durant une procédure d'ablation :
- acquisition de valeurs de mesure fournie par un capteur (11, 13, 15, 17, 19, 21) disposé sur un corps allongé (5) d'une aiguille d'ablation chirurgicale (3) selon la revendication 1 ;
- fourniture d'informations physiologiques du tissu (27, 29, 31) entourant un site d'ablation (33) sur la base des valeurs de mesure acquises.

12. Élément de programme informatique 11, dans lequel les processus commandés comprennent en outre :
- la commande d'un élément d'ablation (9) fourni sur le corps (5) sur la base des informations physiologiques.

13. Support lisible par ordinateur avec un élément de programme informatique selon la revendication 12.
